(19) Europäisches Patentamt
European Patent Office
Office européen des brevets

(11) Veröffentlichungsnummer : **0 132 703 B1**

(12) # EUROPÄISCHE PATENTSCHRIFT

(45) Veröffentlichungstag der Patentschrift : **30.12.86**

(51) Int. Cl.⁴ : **C 07 C 57/04**, C 07 C 93/193, C 07 C103/70, C 07 C 51/42

(21) Anmeldenummer : **84108165.6**

(22) Anmeldetag : **12.07.84**

(54) **Verfahren zur Isolierung von salzförmigen Vinylverbindungen in fester Form aus wässrigen Lösungen.**

(30) Priorität : **20.07.83 DE 3326117**
**19.05.84 DE 3418664**

(43) Veröffentlichungstag der Anmeldung : **13.02.85 Patentblatt 85/07**

(45) Bekanntmachung des Hinweises auf die Patenterteilung : **30.12.86 Patentblatt 86/52**

(84) Benannte Vertragsstaaten : **DE FR GB**

(56) Entgegenhaltungen :
**DE-A- 1 443 694**
**DE-A- 3 011 306**
**DE-A- 3 224 928**
**GB-A- 1 353 212**

(73) Patentinhaber : **Röhm GmbH**
**Kirschenallee Postfach 4242**
**D-6100 Darmstadt 1 (DE)**

(72) Erfinder : **Arndt, Peter Joseph, Dr.**
**Im Säbchen 9**
**D-6104 Seeheim-Jugenheim (DE)**
Erfinder : **Wenzel, Franz, Dr.**
**Zeyherweg 5**
**D-6100 Darmstadt (DE)**
Erfinder : **Müller, Manfred**
**Schillerstrasse 55**
**D-6101 Rossdorf 1 (DE)**
Erfinder : **Schlosser, Fritz**
**Parkstrasse 48**
**D-6100 Darmstadt (DE)**

**EP 0 132 703 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gebiet der Erfindung

Die Erfindung betrifft ein Verfahren zur Isolierung von salzförmigen Vinylverbindungen, insbesondere polymerisationsfähigen Verbindungen dieses Typs, in fester Form aus wäßrigen Lösungen.

Stand der Technik

In der Technik finden verschiedene Typen von salzförmigen Vinylverbindungen Verwendung, hauptsächlich als Monomere zur Herstellung von Polymerisaten bzw. Copolymerisaten. So baut man quartäre Ammoniumgruppen mit Vorliebe in solche Molekeln ein, die wasserlöslich sein sollen.

Von besonderem Interesse sind radikalisch polymerisationsfähige (vinylische) quartäre Ammoniumverbindungen, insbesondere die von Vinylcarbonsäuren, wie der Acryl- bzw. der Methacrylsäure abgeleiteten Verbindungen (vgl. W.F. Hoover in Macromol. Sci. Chem., A4 (6) pp 1327-1417, October 1970). Die Anwendungsgebiete für die daraus gewonnenen kationischen quartären Polyelektrolyte reichen von Flockungs-, Sedimentations- und Verdickungsmitteln bis zu Haarspray-Additiven (Hoover, loc. cit.).

Das den monomeren quartären Ammoniumsalzen zugehörige Anion wird in der Regel durch das Quarternierungsreagenz geliefert. Die Quartenierung von Dimethylaminoäthylmethacrylat mit Dimethylsulfat ist zum Beispiel Gegenstand der US-PS 2 677 699.

Auch in der US-PS 2 741 568 wird die Quarternierung einer über 50 %igen wäßrigen Lösung von Diäthylaminoäthylacrylat mit Dimethylsulfat bei 30-35 °C beschrieben. Die Verwendung der mittels Dialkylsulfat, insbesondere Dimethylsulfat gewonnenen quartären Ammoniumderivate der Acryl- bzw. der Methacrylsäure überwiegt, jedoch sind auch die Halogenide dieser Verbindungsklasse zur Herstellung von Polymeren verwendet worden. (Vgl. G.C. Overberger et al. in J. Polym. Science XXVII, 381-390, 1958). Die Polymerisation derartiger Monomerer in wäßriger Lösung ist eindeutig günstiger als in anderen Lösungsmitteln, weil Übertragungsreaktionen, die sich negativ auf die Molmasse der Polymeren auswirken, hier weniger häufig sind.

Aus DE-OS 20 09 748 ist bekannt, daß ein besonders günstiger Verlauf der Polymerisation zu erwarten ist, wenn man von konzentrierten wäßrigen Lösungen der Monomeren ausgehen kann.

Zum Teil kann man durch Anwendung hochkonzentrierter (wäßriger) Lösungen die Schwierigkeiten umgehen, die eine Aufkonzentrierung aus den Lösungsmitteln mit sich bringt, da sich selbst die Isolierung der aus den quartären Ammoniumsalzen gebildeten Polymerisate aus wäßriger Lösung technisch aufwendig gestaltet, z. B. durch azeotrope Wasserauskreisung mit Hilfe inerter organischer Lösungsmittel oder unter Verwendung von Trockenwalzen (US-PS 3 714 136).

Unmittelbar polymerisationsfähige Mischungen aus Acrylamid und Quarternierungsprodukten von tertiären Aminoalkylestern oder tert. Aminoalkylamiden der Acryl- oder Methacrylsäure erhält man gemäß der DE-PS 2 848 627 in Abwesenheit von Wasser, d. h. ohne Verwendung von Lösungsmitteln. In der US-PS 2 980 657 wird die Quaternierung in Gegenwart von absol. Ethanol als Lösungsmittel vorgenommen ; nach der JP-OS 52-148018 werden gesättigte oder ungesättigte aliphatische Nitrile als Lösungsmittel verwendet.

Außer den quartären Ammoniumsalzen, die sich von aminsubstituierten Estern bzw. Amiden der Acryl- und der Methacrylsäure ableiten, finden auch Metall- und Ammoniumsalze von Vinylcarbonsäuren wie der Acrylsäure und der Methacrylsäure technische Anwendung. So wird in der DE-PS 27 26 260 ein schäumbares Polymermaterial beschrieben, das als Einheiten ein Metallsalz der Acryl- bzw. Methacrylsäure enthält. Gennant werden die $Mg^{2+}$, $Zr^{4+}$, $Cr^{3+}$, $Co^{2+}$, $Zn^{2+}$, $Cd^{2+}$, $Bi^{3+}$, $TiO^{2+}$, oder $Pb^{2+}$-Salze der Acryl- und/oder Methacrylsäure. Ionisch vernetzte Acrylkunststoffe, die durch radikalische Polymerisation eines Monomerengemisches aus mindestens 50 Gew.-% (Meth)acrylsäureestern und 0,1-50 Gew.-% mindestens eines Salzes der Acryl- und/oder Methacrylsäure mit einem Metallkation von einem der Elemente Zink, Aluminium, Zinn, Blei, Titan, Zirkon und Hafnium hergestellt worden sind, bilden den Gegenstand der DE-OS 29 43 566. Ionisch vernetzte Mischpolymerisate aus Methylmethacrylat und Metallsalzen der Methacrylsäure werden in der US-PS 3 493 550 beschrieben. In der DE-OS 29 43 566 wird darauf hingewiesen, daß in der Mehrzahl der Fälle die Metallsalze der Acryl- und der Methacrylsäure schwer darstellbar sind.

In der Regel stellt die Polymerisationstendenz der Säuren und der Salze einen limitierenden Faktor dar. Meist wird von den freien Säuren ausgegangen, die in an sich üblicher Weise mit geeigneten (basischen) Verbindungen der Metalle umgesetzt werden. Wird Wasser als Reaktionsmedium verwendet, so fallen die Salze oft erst nach dem Einengen und im allgemeinen wasserhaltig an. Man bemüht sich z. B. die Isolierung der besonders zur Polymerisation neigenden Metallsalze dadurch zu umgehen, daß man die ungesättigten Säuren zusammen mit basischen Metallverbindungen in den Polymerisationsansätzen verwendet (DE-OS 31 14 266). In der DE-OS 32 24 928 wird daher ein Verfahren zur Herstellung von Metallsalzen der Methacryl- oder der Acrylsäure durch Umsetzung der Säuren mit den Oxiden oder Hydroxiden der Metalle der I., II. und IV. Haupt- und Nebengruppe sowie der VIII.

Nebengruppe in höchstens zweiwertiger Oxidationsstufe beschrieben, bei dem man die Metalloxide bzw. -hydroxide ohne Zusatz eines Lösungsmittels mit mindestens dem 1,5-fachen der stöchiometrisch benötigten Menge an Methacryl- bzw. Acrylsäure umsetzt. Auch die Umsetzung von Metallcarbonaten mit Acryl- bzw. Methacrylsäure im Überschuß ohne Verwendung von Lösungsmitteln ist möglich (DE-OS 32 24 927).

Die Herstellung der Ammoniumsalze der Acryl- oder der Methacrylsäure durch Umsetzung der in wäßrigem Milieu befindlichen säuren mit gasförmigem Ammoniak, wobei während der Reaktion und der nachfolgenden Aufarbeitung die Raumtemperatur nicht wesentlich überschritten werden soll, ist Gegenstand der DE-OS 32 02 663.

Aufgabe

Die Bemühungen des Standes der Technik laufen i. a. darauf hinaus, die hauptsächlich aus der hohen Polymerisationstendenz bzw. sonstigen Reaktivität von salzförmigen α-ständig aktivierten Vinylverbindungen herrührenden Schwierigkeiten dadurch zu umgehen, daß man die Parameter der Umsetzung auf eine möglichst geringe Polymerisationstendenz hin ausrichtet, wobei die Mindestanforderungen an die Produkte wie Reinheit und Einheitlichkeit erfüllt sein müssen. Dazu gehört z. B. das Arbeiten bei höchstens Raumtemperatur, wenn die Salze aus wäßriger Lösung gewonnen werden sollen. Vorliegende Erfahrungen zeigen, daß das azeotrope Wasserauskreisen mit Hilfe inerter organischer Lösungsmittel die Gefahr der Polymerisation keineswegs auf das erforderliche Maß reduziert.

Verschiedene Vorschläge tendieren im übrigen eher dazu, die Herstellungsverfahren langwieriger, aufwendiger und komplizierter zu gestalten als für die an sich einfachen Umsetzungen angemessen erscheint. Es bestand daher nach wie vor das Bedürfnis nach einem Verfahren, das die Herstellung und Isolierung von salzförmigen Vinylverbindungen, insbesondere die Salze und salzförmigen Derivate von Vinylcarbonsäuren aus wäßrigen Lösungen in einfacher, zeit- und kostengünstiger Weise gestattet, wobei die Mindestanforderungen an die Reinheit und Einheitlichkeit der Produkte erfüllt sein müssen.

Lösung

Zur Lösung der bestehenden Aufgabe wird ein Verfahren zur Isolierung von salzförmigen Vinylverbindungen in fester Form aus wäßrigen Lösungen derselben vorgeschlagen, bei dem man die wäßrigen Lösungen der salzförmigen Vinylverbindungen der Sprühtrocknung unterwirft. (Das Vorliegen der Vinylverbindungen in Salzform setzt — wie üblich — die Fähigkeit zur Dissoziation in wäßriger Lösung in von Wasserstoff verschiedene Kationen und in Anionen voraus. Gegebenenfalls können auch Betaine vorliegen).

Unter salzförmigen Vinylverbindungen im Sinne der vorliegenden Erfindung seien vorwiegend die technisch, insbesondere als Monomere für Polymerisationsreaktionen eingesetzten Verbindungen verstanden. Gegenstand des vorliegenden Verfahrens ist insbesondere die Isolierung von Salzen und salzförmigen Derivaten von Vinylcarbonsäuren, speziell der Acryl- und Methacrylsäure, der Maleinsäure, der Fumarsäure und der Itaconsäure. Das erfindungsgemäße Verfahren kommt somit in erster Linie für die technisch verwendbaren Salze bzw. salzförmigen Verbindungen der Vinylcarbonsäuren in Frage, z. B. für die im Stande der Technik genannten (vgl. W.F. Hoover loc. cit. und Patentschriften). Bei der Durchführung des erfindungsgemäßen Verfahrens liegen die Salze bzw. die salzförmigen Derivate in wäßriger Lösung, gegebenenfalls auch in einem Gemisch aus Wasser und organischen, wassermischbaren Lösungsmitteln, wie etwa Alkoholen, Aceton u. ä., vor. Die vorliegende Aufgabe stellt sich hauptsächlich, wenn eine hinreichende Wasserlöslichkeit der salzförmigen Vinylverbindungen vorliegt. Als eine hinreichende Wasserlöslichkeit wird eine Löslichkeit von mindestens 20 g des Salzes in 100 g Wasser bei 20 °C angesehen. Von besonderem Interesse sind die Salze der (Meth)acrylsäure, daneben auch die der Maleinsäure, Fumarsäure und Itaconsäure. Insbesondere seien genannt die Alkalisalze, speziell die Natrium-, Kalium-, Lithiumsalze, die Erdalkalisalze, insbesondere die Magnesium-, Calcium-, Strontium- und Bariumsalze; die Salze der I. und II. Nebengruppe des periodischen Systems, insbesondere die Kupfer- und Zinksalze. Ferner die Salze der IV. Haupt- und Nebengruppe, insbesondere Germanium-, Zinn-, Blei-, Titan-, und Zirkonsalze, weiter die Salze der VIII. Nebengruppe, insbesondere Eisen-, Kobalt- und Nickelsalze, ferner die Salze der V. und VII. Haupt- und Nebengruppe, insbesondere Antimon-, Wismut-, Vanadin- und Mangansalze, weiter die Ammoniumsalze, sowohl die von Ammoniumkationen der Formel

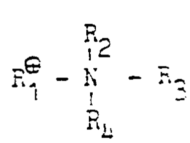

abgeleitet sind, worin $R_1$ und $R_2$ für Wasserstoff oder einen Alkylrest mit 1 bis 18, vorzugsweise 1 bis 5 Kohlenstoffatomen, $R_3$ und $R_4$ für Wasserstoff oder einen Alkylrest mit 1 bis 18, vorzugsweise 1 bis 6

Kohlenstoffatomen oder worin $R_3$ und $R_4$ gegebenenfalls unter Einbeziehung eines oder zweier weiterer Heteroatome wie Stickstoff, Sauerstoff oder Schwefel ein fünf- oder sechsgliedriges heterocyclisches System bilden.

| Genannt seien speziell : | Löslichkeit in $H_2O$ bei 20 °C in g Salz/100 g $H_2O$ |
|---|---|
| Kaliummethacrylat | 101,8 |
| Natriummethacrylat | 95,3 |
| Lithiummethacrylat | 87,5 |
| Ammoniummethacrylat | 104,5 |
| Strontiumdimethacrylat | 27,5 |
| Bariumdimethacrylat | 40,5 |
| Natriumacrylat | 49,0 |
| Kaliumacrylat | 166,5 |
| Ammoniumacrylat | 242,0 |
| Strontiumdiacrylat | 68,0 |
| Calciumdiacrylat | 35,0 |
| Lithiumacrylat | 45,0 |
| Bariumdiacrylat | 74,0 |
| Zinkdiacrylat | 40,0 |
| Dinatriumitaconat | 66,7 |
| Dinatriummaleinat | 96,0 |
| Dinatriumfumarat | 22,8 |

Weiterhin sind bevorzugt als salzförmige Vinylverbindungen die quartären Ammoniumsalze der Formel I

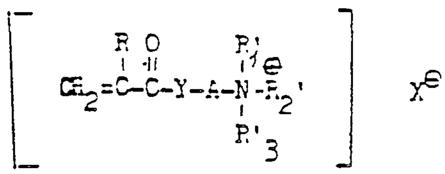

(I)

worin R Wasserstoff oder Methyl, Y Sauerstoff oder $-NR_4$, wobei $R_4$ für Wasserstoff oder einen Alkylrest mit 1 bis 6 Kohlenstoffatomen steht, A eine gegebenenfalls verzweigte Kohlenwasserstoffbrücke mit 2 bis 8 Kohlenstoffatomen, $R'_1$ und $R'_2$ unabhängig voneinander Alkylreste mit 1 bis 6 Kohlenstoffatomen und $R'_3$ einen Alkylrest mit 1 bis 6 Kohlenstoffatomen oder einen Benzylrest oder einen Rest $-CH_2-CH=CH_2$, $-CH_2-O-CH_3$, $-CH_2-CH_2-OH$ bedeutet und X für das Anion aus dem quaternierenden Reagenz steht.

Das erfindungsgemäße Verfahren ist im Prinzip auf wäßrige Lösungen quartärer Ammoniumsalze von Vinylverbindungen ohne Unterschied anwendbar. Dazu gehören neben den Verbindungen der Formel I quartäre Diallyl- und Allylammoniumsalze, Vinyloxyalkylammoniumsalze, Vinylbenzylammonium- und Vinylaniliniumsalze u. a.

Besonders genannt seien die Ester und die Amide der Formel I in denen R für Methyl steht, d. h. quartäre Ammoniumsalze als Abkömmlinge der Methacrylsäure, daneben die der Acrylsäure. Ferner sind die Verbindungen der Formel I bevorzugt, bei denen A für einen Rest $-(CH_2)_2-$, $-(CH_2)_3-$ oder $-CH_2-C(CH_3)_2-CH_2-$ steht. Im weiteren sind von besonderem Interesse die Verbindungen bei denen $R'_1$, $R'_2$, $R'_3$ für Methyl oder Äthyl stehen. Als Anionen sind — unter technischen Gesichtspunkten — vor allem das Chlorid- und daneben das Methylsulfatanion wichtig. Genannt seien z. B. das Trimethylammoniumäthylmethacrylatchlorid und die entsprechende Triethylammoniumverbindung.

Für das Verfahren ist die Konzentration des Festproduktes in der wäßrigen Lösung innerhalb eines weiten Bereiches nicht kritisch. Vom Herstellungsverfahren der quartären Ammoniumsalze her werden in der Regel hochprozentige Lösungen angestrebt, etwa im Bereich 50-85 Gew.-% Festprodukt in der wäßrigen Lösung.

Im Interesse einer günstigen Energiebilanz wird man in allen Fällen bemüht sein, relativ konzentrierte Salzlösungen im erfindungsgemäßen Verfahren einzusetzen. Ein geringerer Salzgehalt wirkt zwar in der Regel technisch nicht limitierend, wohl aber in ökonomischer Hinsicht.

In allgemeinen liegt der Salzgehalt der erfindungsgemäß zu verwendenden Lösungen innerhalb der Grenzen von 20 Gew.-% bis zur Sättigungskonzentration. Als Richtwert für Acrylate seien z. B. 35 Gew.-% angegeben. Die Herstellung der wäßrigen Salzlösungen von z. B. Vinylcarbonsäuren wird in an sich bekannter Weise z. B. durch Neutralisation der Säuren mit Oxiden, Hydroxiden, Alkoxiden, Carbonaten, Bicarbonaten derjenigen Metalle bzw. Ammoniumverbindungen durchgeführt, deren Salze man gewinnen will oder gegebenenfalls auch mit Salzen von sonstigen, flüchtigen, insbesondere schwachen Säuren. Mit Vorteil führt man die Herstellung der Salze in wäßrigem Milieu so durch, daß die entstehenden wäßrigen Lösungen unmittelbar der erfindungsgemäßen Verarbeitung durch Sprühtrocknung zugeführt werden können. Hierbei und bei der Durchführung des erfindungsgemäßen Verfahrens empfiehlt sich der Zusatz an sich bekannter Stabilisatoren bzw. Polymerisationsinhibitoren wie z. B. Chinone, Phenole, Methylenblau, aromatische Amine (Diphenylamin) oder Nitroverbindungen gegebenenfalls auch Kupfer- oder Eisensalze in den üblicherweise verwendeten Mengen. Die zuzusetzenden Mengen liegen in der Regel bei 0,01 bis 0,1 Gew.-%, bezogen auf die stabilisierenden monomeren Vinylverbindungen. Genannt seien beispielsweise der Hydrochinonmonomethylether, das 4-Methyl-2,6-di-tert.Butylphenol (vgl. Houben-Weyl, 4. Auflage, Band XIV/1, S. 42, 1011, Georg-Thieme-Verlag, 1961).

Im Interesse eines möglichst einheitlichen Produkts wird man beispielsweise von der Verwendung von Überschüssen an Reaktanten bei der Herstellung der Salze absehen. z. B. wird man zur Neutralisation etwa stöchiometrische Mengen der das Kation beisteuernden base anwenden.

Es können selbstverständlich auch aus anderem Anlaß anfallende wäßrige Lösungen von Salzen der Vinylcarbonsäuren dem erfindungsgemäßen Verfahren unterworfen werden. Unter Sprühtrocknung oder Zerstäubungstrocknung wird im Einklang mit der üblichen Terminologie die Zerteilung flüssiger Trockengüter in feine, nebelartige Tröpfchen und die Trocknung, gewöhnlich mit einem heißen Luftstrom, verstanden. (Vgl. Ullmanns Encyklopädie der techn. Chemie, 4. Auflage, Band 2, pg. 712 u. 713, Verlag Chemie, 1972). Als Zerstäubungseinrichtung werden Düsen (Einstoff- oder Zweistoffdüsen) oder rotierende Scheiben mit gewöhnlich einer Umdrehungszahl von 4 000-30 000 U/min verwendet (vgl. Masters Ind. Eng. Chemistry, *60*, (1968) Nr. 10, S. 53-63). Die Lufteintrittstemperatur liegt zweckmäßig im Bereich 100-200 °C, die Austrittstemperatur bei 50-100 °C, vorzugsweise 90-100 °C. Die Verweilzeiten betragen ca. 0,1-30 sec., vorteilhaft 0,5-10 sec. Das erfindungsgemäße Verfahren sei im folgenden näher erläutert :

Zur Herstellung der Salzlösung

wird zweckmäßig in einem geeigneten rührbaren und kühlbaren Kessel die gewünschte Menge Wasser zusammen mit der $\alpha$,$\beta$-ungesättigten Carbonsäure und dem Stabilisator vorgelegt und unter Lufteinleitung die basische Metallverbindung portionsweise zugegeben. Vorteilhaft sorgt man, gegebenenfalls durch Kühlung dafür, daß die Temperatur nicht über 30 °C ansteigt. Nachdem das gesamte Gut in Lösung gegangen ist, wird über einen geeigneten Filter filtriert. Man erhält in der Regel eine klare Lösung mit dem vorbestimmten Salzgehalt.

Die Sprühtrocknung

der salzartigen Vinylverbindungen kann mit den entsprechenden Sprüh- bzw. Zerstäubungstrocknungsvorrichtungen des Standes der Technik durchgeführt werden. Beispielsweise können mit Vorteil Scheibensprühtürme mit zwischen 4 000 und 30 000, vorzugsweise 22 000 ± 5 000 UpM verwendet werden. Vorteilhaft ist z. B. die Verwendung von geschlossenen Scheiben. In vielen Fällen empfiehlt es sich eine möglichst feine Zerstäubung des Sprühguts anzustreben, indem man im oberen möglichen Drehzahlbereich arbeitet. Die Lufteintrittstemperatur liegt im Bereich 100 bis 200 °C, zweckmäßig bei 192 ± 5 °C, die Austrittstemperatur bei 50 bis 100 °C, vorzugsweise bei 90 ± 5 °C. Die Verweilzeiten betragen ca. 0,1-30 sec. Vorteilhaft 0,5-10 sec., insbesondere 7 ± 2 sec. Die zugeführte Trockenluftmenge kann als Anhalt ca. 400-20 000 $m^3$/h betragen. Als Dosiereinrichtung kann eine Schlauchpumpe verwendet werden. Das Absieben kann über ein geeignetes Sieb, beispielsweise ein 0,8 mm Sieb (Typ : Allgaier-Sieb) vorgenommen werden.

## Beispiel 1

Ca. 20 kg einer ca. 35 %igen Zinkdiacrylatlösung werden in einem Scheibensprühturm, ausgerüstet mit einer Normalscheibe mit 20 000 UpM ; Lufteintrittstemperatur 190 °C, Luftaustrittstemperatur 91-95 °C sprühgetrocknet. Die Trockenluftmenge beträgt ca. 400 $m^3$/h. Die Verweilzeiten betragen 5 bis 10 sec. Der Durchsatz beträgt 55,5 kg Zinkdiacrylatlösung (135 %ig) pro 158 min entsprechend ca. 21,1 kg Zinkdiacrylatlösung pro Stunde. Die Sprühausbeute liegt bei über 84 %. Es werden klare, z. T. verglaste Perlen gebildet. Ihr Hauptanteil liegt bei 10-20 $\mu$, die größten Partikel sind ca. 80 $\mu$ groß. Die Restfeuchte des Sprühguts liegt bei 0,35-0,55 %, das Schüttgewicht bei 540-600 g/l. Durch Erhöhung der Arbeittemperatur kann man höhere Schüttgewichte erzielen. Die Sprühanlage läßt sich infolge der guten Wasserlöslichkeit der Sprühprodukte relativ leicht reinigen. Eine Polymerisation zu messbaren Anteilen wurde nicht beobachtet. In analoger Weise können auch die Salze der Metalle der I., II., IV., V. und VII. Haupt- und Nebengruppe und der VIII.-Nebengruppe sowie Ammoniumsalze isoliert werden, insbesondere die $K^+$, $Na^+$, $Li^+$, $Sr^{2+}$, $Ba^{2+}$, $Sn^{2+}$ und Ammoniumsalze.

Das folgende Beispiel betrifft die Anwendung des Verfahrens auf quartäre Ammoniumsalze.

## Beispiel 2

Ausgehend von Dimethylaminoethylmethacrylat, Methylchlorid, Wasser und Stabilisator wurde durch Druckquaternierung eine im Feststoffgehalt ca. 80 %ige wäßrige Trimethylammoniumethylmethacrylatchloridlösung erhalten, die für die Sprühtrocknung eingesetzt wird. Für die Sprühtrocknung stand ein Scheibensprühturm der Fa. NIRO des Typs MINOR-PRODUCTION mit einer max. Wasserverdampfung von ca. 30 kg/h Wasser, einem Luftdurchsatz von ca. 400 $m^3$/h, einer Lufteintrittstemperatur von ca. 250 °C und einer regelbaren Scheibendrehzahl bis ca. 20 000 UpM zur Verfügung. Auf die mit ca. 15 000 UpM rotierende Lochscheibe wurden bei Heißlufteingangstemperaturen von 125 ± 25 °C kg/h Trimethylammoniumethylmethacrylat-chloridlösung aufgegeben. Die Ablufttemperatur betrug 80 ± 10 °C. In ca. 95 %iger Ausbeute konnten aus dem dem Sprühtrockner nachgeschalteten Zyklon ca. 15 kg/h festes Trimethylammoniummethylmethacrylatchlorid mit einem Restwassergehalt von < 1 % gewonnen werden. Das Sprühprodukt ist polymerisatfrei und eignet sich hervorragend zur Herstellung von hochmolekularen Polymerisaten als Flockungsmittel. So wurde durch Polymerisation in 75 %iger wäßriger Lösung ein hochmolekulares Polymers erhalten, das 1 %ig in Wasser Viskositäten von 7 000 mPa · s ergab und hervorragende Flockungseigenschaften aufwies.

In analoger Weise können auch die weiteren quartären Ammoniumsalze der Formel I hergestellt werden, z. B. das Trimethylammoniumpropylacrylat-chlorid oder methosulfat, das Benzyldimethylammoniumethylacrylat-chlorid, das Dimethylallylammoniumpropylacrylat-chlorid, das Dimethylmethoxymethylammoniumethylacrylat-chlorid, das β-Hydroxyethyldimethylammoniumpropylacrylat-bromid und die entsprechenden Methacrylsäureester sowie das N-[3-(Trimethylammonium)-2,2-dimethylpropylmethacrylamid-tosylat].

## Beispiel 3

Ca. 40 kg einer ungefähr 45 %igen wäßrigen Lösung von Natriummethacrylat werden in einem Scheibensprühturm, ausgerüstet mit einer Normalscheibe mit 20 000 UpM, Lufteintrittstemperatur 185 °C und einer Luftaustrittstemperatur zwischen 85 und 90 °C sprühgetrocknet. Die Trockenluftmenge beträgt ca. 400 $m^3$/h. Die Verweilzeit beträgt zwischen 7 und 12 sec. Der Durchsatz beträgt 23,7 kg Natriummethacrylat-Lösung (45 %ig) pro Stunde. Die Sprühausbeute liegt bei 10,3 kg Festsubstanz pro Stunde entsprechend 96,6 % Ausbeute. Man erhält klare Perlen im Bereich 10 bis 100 μ. Die Restfeuchte des Sprühguts liegt bei weniger als 0,3 Gew.-%. Das so erhaltene Produkt ist sofort wasserlöslich und ist im wesentlichen frei von Polymerisat. In analoger Weise können auch die weiteren Salze der Methacrylsäure, beispielsweise der I., II., IV., V. und VII. Haupt- und Nebengruppe, der VIII. Nebengruppe sowie Ammoniumsalze isoliert werden, insbesondere die $K^{\oplus}$, $Li^{\oplus}$, $Sr^{2+}$, $Ba^{2+}$, $Sn^{2+}$ und die Ammoniumsalze.

## Beispiel 4

Das Dinatriumsalz der Itaconsäure in einer 40 %igen wäßrigen Lösung wird durch Umsetzung von Itaconsäure in Wasser mit 2 Mol Natriumhydroxid hergestellt. Nach dem Filtern erhält man eine völlig klare Lösung. Die Durchführung der Sprühtrocknung kann in weitgehender Anlehnung an Beispiel 3 erfolgen. Der Durchsatz beträgt 23,3 kg Dinatrium-Itaconat (40 %ig, Schmelzpunkt 290-300 °C). Man erhält 8,3 kg Festsubstanz pro Stunde entsprechend 89 % Ausbeute. In ähnlicher Weise können auch die anderen Salze von Vinyldicarbonsäuren, z. B. die Natrium-, Kalium- und Ammoniumsalze der Maleinsäure und der Fumarsäure hergestellt werden.

## Patentansprüche

1. Verfahren zur Isolierung von salzförmigen Vinylverbindungen in fester Form aus wäßrigen Lösungen derselben, dadurch gekennzeichnet, daß man die wäßrigen Lösungen der salzförmigen Vinylverbindungen der Sprühtrocknung unterwirft.

2. Verfahren gemäß Anspruch 1, dadurch gekennzeichnet, daß als salzförmige Vinylverbindungen solche eingesetzt werden, die als Monomere für Polymerisationen eingesetzt werden.

3. Verfahren gemäß Anspruch 2, dadurch gekennzeichnet, daß als Salze der Vinylcarbonsäuren solche der Acrylsäure, der Methacrylsäure, der Maleinsäure, der Fumarsäure oder der Itaconsäure eingesetzt werden.

4. Verfahren gemäß den Ansprüchen 1 bis 3, dadurch gekennzeichnet, daß die Salze der Metalle der I., II., IV., V. und VII. Haupt- und Nebengruppe und der VIII. Nebengruppe sowie Ammoniumsalze verwendet werden.

5. Verfahren gemäß den Ansprüchen 2 bis 4, dadurch gekennzeichnet, daß die Alkali-, Erdalkali- und Ammoniumsalze verwendet werden.

6. Verfahren gemäß den Ansprüchen 2 bis 4, dadurch gekennzeichnet, daß die Zinksalze verwendet werden.

7. Verfahren gemäß den Ansprüchen 1 und 2, dadurch gekennzeichnet, daß als salzförmige Vinylverbindungen quartäre Ammoniumsalze der Formel I

(Siehe Formel I Seite 7 f.)

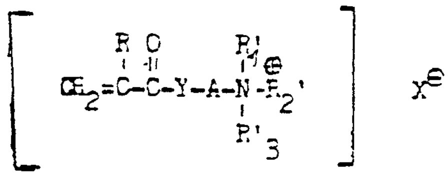

(I)

worin R Wasserstoff oder Methyl, Y Sauerstoff oder $—NR_4$, wobei $R_4$ für Wasserstoff oder einen Alkylrest mit 1 bis 6 Kohlenstoffatomen steht, A eine gegebenenfalls verzweigte Kohlenwasserstoffbrücke mit 2 bis 8 Kohlenstoffatomen, $R'_1$ und $R'_2$ unabhängig voneinander Alkylreste mit 1 bis 6 Kohlenstoffatomen und $R'_3$ einen Alkylrest mit 1 bis 6 Kohlenstoffatomen, einen Benzylrest oder einen Rest $—CH_2—CH=CH_2$, $—CH_2—O—CH_3$, $—CH_2—CH_2—OH$ bedeutet und X für das Anion aus dem quaternierenden Reagenz steht, eingesetzt werden.

8. Verfahren gemäß Anspruch 7, dadurch gekennzeichnet, daß $X^{\ominus}$ in Formel I für ein Chlorid, Bromid, Jodid, Methylsulfat, Tosylat-Anion steht.

9. Verfahren gemäß den Ansprüchen 1 bis 8, dadurch gekennzeichnet, daß man salzförmige Vinylverbindungen einsetzt, deren Wasserlöslichkeit mindestens 20 g in 100 g Wasser bei 20 °C beträgt.

10. Verfahren gemäß den Ansprüchen 1 bis 9, dadurch gekennzeichnet, daß die wäßrige Lösung mindestens 20 Gew.-% Festprodukt enthält.

11. Verfahren gemäß den Ansprüchen 1 bis 10, dadurch gekennzeichnet, daß die Lufteintrittstemperatur bei der Sprühtrocknung 100-200 °C beträgt.

12. Verfahren gemäß den Ansprüchen 1 bis 11, dadurch gekennzeichnet, daß die Austrittstemperatur bei der Sprühtrocknung 50-100 °C beträgt.

## Claims

1. Process for isolating saliniform vinyl compounds in solid form from aqueous solutions thereof, characterised in that the aqueous solutions of the saliniform vinyl compounds are subjected to spray drying.

2. Process as claimed in claim 1, characterised in that the saliniform vinyl compounds used are those which are used as monomers for polymerisation processes.

3. Process as claimed in claim 2, characterised in that the salts of vinylcarboxylic acids used are the salts of acrylic, methacrylic, maleic, fumaric or itaconic acid.

4. Process as claimed in claims 1 to 3, characterised in that the salts of metals of the Ist, IInd, IVth, Vth and VIIth main and sub-groups and the VIIIth sub-group and ammonium salts are used.

5. Process as claimed in claims 2 to 4, characterised in that the alkali metal, alkaline earth metal and ammonium salts are used.

6. Process as claimed in claims 2 to 4, characterised in that the zinc salts are used.

7. Process as claimed in claims 1 and 2, characterised in that the saliniform vinyl compounds used are quaternary ammonium salts of formula I

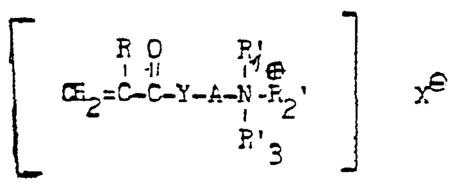

(I)

wherein R represents hydrogen or methyl, Y represents oxygen or $—NR_4$, wherein $R_4$ is hydrogen or an alkyl group with 1 to 6 carbon atoms, A represents an optionally branched hydrocarbon bridge with 2 to 8 carbon atoms, $R'_1$ and $R'_2$ independently of each other represent alkyl groups with 1 to 6 carbon atoms and $R'_3$ represents an alkyl group with 1 to 6 carbon atoms, a benzyl group or a group $—CH_2—CH=CH_2$, $—CH_2—O—CH_3$, $—CH_2CH_2—OH$ and X represents the anion from the quaternising reagent.

8. Process as claimed in claim 7, characterised in that $X^{\ominus}$ in formula I represents a chloride, bromide, iodide, methylsulphate or tosylate anion.

9. Process as claimed in claims 1 to 8, characterised in that saliniform vinyl compounds are used the water solubility of which is at least 20 g in 100 g of water at 20 °C.

10. Process as claimed in claims 1 to 9, characterised in that the aqueous solution contains at least 20 % by weight of solid product.

11. Process as claimed in claims 1 to 10, characterised in that the air inlet temperature for spray drying is 100 to 200 °C.

12. Process as claimed in claims 1 to 11, characterised in that the outlet temperature for the spray drying is 50 to 100 °C.

**Revendications**

1. Procédé d'isolement de composés vinyliques sous forme de sels à l'état solide à partir de solutions aqueuses, caractérisé en ce qu'on soumet à un séchage par pulvérisation les solutions aqueuses de composés vinyliques sous forme de sels.

2. Procédé selon la revendication 1, caractérisé en ce qu'on traite, en tant que composés vinyliques sous forme de sels, des composés qui sont utilisés comme monomères pour des polymérisations.

3. Procédé selon la revendication 2, caractérisé en ce qu'on traite, en tant que sels des acides vinylcarboxyliques, des sels de l'acide acrylique, de l'acide méthacrylique, de l'acide maléique, de l'acide fumarique ou de l'acide itaconique.

4. Procédé selon l'une quelconque des revendications 1 à 3, caractérisé en ce qu'on utilise les sels des métaux des groupes principaux et auxiliaires I, II, IV, V et VII et du groupe auxiliaire VIII, ainsi que des sels d'ammonium.

5. Procédé selon l'une quelconque des revendications 2 à 4, caractérisé en ce qu'on utilise les sels alcalins, alcalinoterreux et les sels d'ammonium.

6. Procédé selon l'une quelconque des revendications 2 à 4, caractérisé en ce qu'on utilise les sels de zinc.

7. Procédé selon la revendication 1 ou 2, caractérisé en ce qu'on traite, en tant que composés vinyliques sous forme de sels, des sels d'ammonium quaternaires de formule I

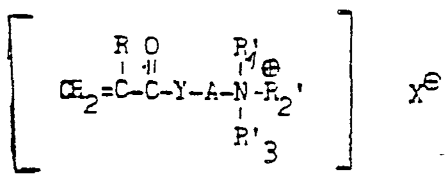

(I)

dans laquelle R représente un atome d'hydrogène ou un radical méthyle, Y représente un atome d'oxygène ou un groupement $—NR_4$, $R_4$ étant mis pour un atome d'hydrogène ou un radical alkyle à 1-6 atomes de carbone, A représente un pont hydrocarboné éventuellement ramifié à 2-8 atomes de carbone, $R'_1$ et $R'_2$ représentent, indépendamment l'un de l'autre, des radicaux alkyle à 1-6 atomes de carbone et $R'_3$ représente un radical alkyle à 1-6 atomes de carbone, un radical benzyle ou un radical $—CH_2—CH=CH_2$, $—CH_2—O—CH_3$, $—CH_2—CH_2—OH$ et X est mis pour l'anion du réactif quaternisant.

8. Procédé selon la revendication 7, caractérisé en ce que $X^\ominus$ dans la formule I est mis pour un anion chlorure, bromure, iodure, méthylsulfate ou tosylate.

9. Procédé selon l'une quelconque des revendications 1 à 8, caractérisé en ce qu'on traite des composés vinyliques sous forme de sels dont la solubilité dans l'eau s'élève au moins à 20 g dans 100 g d'eau à 20 °C.

10. Procédé selon l'une quelconque des revendications 1 à 9, caractérisé en ce que la solution aqueuse contient au moins 20 % en poids de produit solide.

11. Procédé selon l'une quelconque des revendications 1 à 10, caractérisé en ce que la température d'entrée de l'air lors du séchage par pulvérisation s'élève à 100-200 °C.

12. Procédé selon l'une quelconque des revendications 1 à 11, caractérisé en ce que la température de sortie lors du séchage par pulvérisation s'élève à 50-100 °C.